# EUROPEAN PATENT SPECIFICATION

(11) **EP 4 299 097 B1**
(45) Date of publication and mention of the grant of the patent: **17.09.2025**
(21) Application number: 23181623.2
(22) Date of filing: 27.06.2023
(51) Int. Cl.: A61M 16/12, A61M 16/00

(54) **SYSTEM FOR CONTROLLING AND MEASURING OXYGEN DELIVERY THROUGH A CPAP ADAPTER**
SYSTEM ZUR STEUERUNG UND MESSUNG DER SAUERSTOFFABGABE DURCH EINEN CPAP-ADAPTER
SYSTÈME DE COMMANDE ET DE MESURE DE L'ADMINISTRATION D'OXYGÈNE PAR L'INTERMÉDIAIRE D'UN ADAPTATEUR CPAP

(30) Priority: 27.06.2022 US 202217850696
(43) Date of publication of application: 03.01.2024
(73) Proprietor: Dynasthetics, LLC, Salt Lake City, UT 84119 (US)
(72) Inventor: ORR, Joseph, Salt Lake City, 84119 (US); BURK, Kyle M., Salt Lake City, 84119 (US); BLACKWELL, Trey, Salt Lake City, 84119 (US)
(74) Representative: Wohlfahrt, Jan Günther

(56) References cited:
- US-A1- 2018 177 961
- US-A1- 2020 368 482

## Description

### TECHNICAL FIELD

The present disclosure relates generally to respiratory assist devices. More specifically, the disclosure relates to a respiratory fluid control device or adapter for delivering and calculating a total volume of gas delivered to a patient.

### BACKGROUND

Positive airway pressure therapies are used for the treatment of various respiratory conditions including both central apnea and obstructive apnea. During sleep or sedation, it is common in patients for the muscles that normally hold the upper airway open to relax so airway tissues partially occlude or obstruct the airway, preventing adequate gas flow to the lungs. In many patients that are in a relaxed state such as sleeping or who have received sedative drugs, the muscles of the upper airways are relaxed to such an extent that any effort to inhale by the patient collapses the airway and obstructs gas flow restricting ability to draw in breathable gas. The negative pressure created as the patient tries to draw gas into the lungs pulls the airway closed even more, exacerbating the problem.

Positive airway pressure (PAP) therapies use a breathing mask and a source of flowing breathable gas to create positive pressure in the airway. This positive pressure supports the airway opening such that it does not collapse as the patient inhales. The flow generator used for PAP must have sufficient flow to supply patient inhalation volume and compensate for any mask leak. The flow generator must also have sufficient positive pressure to counterbalance any negative pressures created by the patient during inhalation. Continuous positive airway pressure, or CPAP, is a specific PAP therapy that continuously applies a specific prescribed pressure to the airways. CPAP forces the airway to stay open during inhalation so that breathable gas can pass freely into the lungs on demand even when the airway opening is relaxed.

It may be desirable to be able to determine the total flow output from a flow generator, and/or to determine an amount of oxygen inhaled by a patient. Prior art systems and methods do not allow clinicians to readily calculate or determine patient tidal volume of oxygen inhaled.
US 2020/368482 A1 discloses a ventilator in communication with an oxygen concentrator, the ventilator comprising a controller configured to calculate a total flow from a measured flow of gas expelled by one or more nozzles of a patient ventilation interface and measured pressure in the patient ventilation interface.
US 2018/177961 A1 discloses a respiratory device for supplying breathing air to a patient, including an oxygen inlet to be connected to an oxygen supply, and a compressed air inlet to be connected to a compressed air supply. The respiratory device has a pressure sensor to measure pressure of the gas mixture at a patient connection port, a flow rate sensor to measure flow rate of the oxygen through an oxygen duct, and a flow rate sensor to measure flow rate of the air in an air duct. A total flow is determined by the sum of the measured pressures and flow rates of the flow rate sensors.

### SUMMARY OF DISCLOSURE

The invention is defined in the independent claim 1. Preferred embodiments are matter of the dependent claims.

According to the present disclosure, a system for determining total flow output from a flow generator can include: the flow generator for connection to a patient interface; the patient interface in communication with a patient interface pressure sensor to measure a patient interface pressure at the patient interface; an oxygen input line for conveying oxygen from a source of oxygen to the flow generator, the oxygen input line comprising an oxygen control valve for controlling a flow of oxygen from an oxygen source to the flow generator, and an oxygen input line sensor for determining oxygen input line flow; and wherein the patient interface pressure sensor and the oxygen input line sensor are in communication with a controller, the controller programmed to receive the patient interface pressure and the oxygen input line flow and determine the total flow output from the flow generator based on the patient interface pressure at the patient interface and the oxygen input line flow.

In some configurations, the oxygen input line sensor comprises a pressure sensor for measuring oxygen input line pressure and wherein the oxygen input line flow is determined by the oxygen input line pressure. In other examples, the oxygen input line sensor comprises an oxygen flowmeter for measuring an oxygen input line flow. The controller can be programmed to determine the total gas flow output from the flow generator based on the patient pressure at the patient interface and the oxygen input line flow. In some examples the controller is programmed with at least one algorithm to calculate total flow output based on known patient pressure at the patient interface and known oxygen input line flow.

In some examples (not separately claimed), a method for determining total flow generator flow output can include the following steps: obtaining, by a processor, information relating to a patient interface pressure and an oxygen input line flow; and determining, by the processor, a total flow generator flow output based on the patient interface pressure and the oxygen input line flow. The method can also include the step of obtaining, by a processor, information relating to a flow generator type; and wherein the step of determining, by the processor, a total flow generator flow output comprises determining, by the processor, the total flow generator flow output based on the patient interface pressure, the oxygen input line flow, and the flow generator type. The controller may be programmed with at least one algorithm to calculate the total flow generator flow output based on known patient interface pressure at the patient interface and known oxygen input line flow.

According to another aspect, a system is described for determining an amount of gas inhaled by a patient. The system can include: a flow generator for connection to a patient interface; the patient interface in communication with a patient interface pressure sensor to measure patient interface pressure at the patient interface; an oxygen input line for conveying oxygen from a source of oxygen to the flow generator, the oxygen input line comprising an oxygen control valve for controlling a flow of oxygen from the source of oxygen to the flow generator, and an oxygen input line sensor for determining an oxygen input line flow; wherein the patient interface pressure sensor and the oxygen input line sensor are in communication with a processor, and wherein the processor is programmed to receive, during a pause between a patient exhalation and a patient inhalation, the patient pressure at the patient interface and the oxygen input line flow and determine a leak flow output from the flow generator based on the patient pressure at the patient interface and the oxygen input line flow during the pause between the patient exhalation and the patient inhalation; wherein the processor is further programmed to receive, during the patient inhalation, the patient pressure at the patient interface and the oxygen input line flow and determine a total flow output from the flow generator based on the patient pressure at the patient interface and the oxygen input line flow during a patient inhalation; and wherein the processor is further programmed to calculate a patient flow by subtracting the leak flow output from the total flow output.

The processor can be programmed to calculate a tidal volume of gas by integrating the patient flow over a single breath. The controller can be programmed with at least one algorithm to calculate total flow output based on known patient pressure at the patient interface and known oxygen input line flow. The controller can further be programmed to calculate a concentration of oxygen in the patient flow to determine a fraction of inhaled oxygen.

According to another aspect, the system further comprises a housing, the controller, the oxygen control valve, and the patient interface pressure sensor located within the housing, the housing having a coupler to couple to the source of oxygen to the housing, and the housing have a receptacle; and wherein the flow generator is coupled to a proximal end of the oxygen input line, and wherein a distal end of the oxygen input line comprises a connector to connect to the receptacle of the housing, the connector comprising a type-identifier, and the receptacle comprising a reader in communication with the controller, the reader to read the type-identifier and convey the type-identifier to the controller.

In yet another aspect, aspect (not separately claimed), a method for calculating patient tidal volume of gas inhaled includes: obtaining, by a processor, information relating to a patient interface pressure and an oxygen input line flow, determining, by the processor, a total flow generator flow output based on the patient interface pressure and the oxygen input line flow, obtaining, by the processor, a leak flow, calculating, by the processor, patient flow by subtracting the leak flow from the total flow generator flow output; calculating, by the processor, patient tidal volume of gas inhaled by integrating the patient flow over a single breath. The step of determining, by the processor, a flow generator total flow output based on the patient interface pressure and the oxygen input line flow, can include the processor determining the flow generator total flow output based on at least one of: a pre-defined mathematical function stored in a memory accessible by the processor, a lookup table stored in a memory accessible by the processor, and a trained artificial neural network stored in a memory accessible by the processor. In some examples, the method further comprises the step of calculating a concentration of oxygen in the patient flow to determine a fraction of inhaled oxygen.

According to another aspect (not separately claimed), a method for calculating a percentage of oxygen in a volume of gas inhaled during an inhalation can include the steps of: obtaining, by a processor, information relating to a flow generator input; obtaining, by a processor, information relating to a flow generator output; determining, by the processor, an oxygen leak flow, calculating, by the processor, a percentage of oxygen in a volume of gas inhaled by: adding 21% of a difference between a total oxygen flow generator flow output and a total oxygen flow generator flow input over a time of the inhalation to the total oxygen flow generator flow input over the time of the inhalation, subtracting the oxygen leak flow over the time of the inhalation to determine a volume of oxygen inhaled; and dividing the volume of oxygen inhaled by a total volume of gas inhaled. The step of determining, by the processor, the oxygen leak flow, comprises determining, by the processor, a pause period between a patient exhalation and a patient inhalation, and wherein the method further comprises the steps of: obtaining, by a processor, the flow generator output during the pause period between the patient exhalation and the patient inhalation; and determining, by the processor, a flow generator leak flow based on the flow generator output during the pause period.

### BRIEF DESCRIPTION OF DRAWINGS

The drawings are illustrative and not limiting of the scope of the invention which is defined by the appended claims. Not every element of the disclosure can be clearly displayed in a single drawing, and as such not every drawing shows each element of the disclosure. The components in the drawings are not necessarily to scale relative to each other. Like reference numerals designate corresponding parts throughout the several views.
FIG. 1 a block diagram of an exemplary system for delivering and/or measuring oxygen delivered to a patient.
FIG. 2 is a block diagram of another exemplary system for delivering and/or measuring delivered oxygen.
FIG. 3 is a block diagram of another exemplary system for delivering and/or measuring delivered oxygen.
FIG. 4 is a block diagram of another exemplary system for delivering and/or measuring delivered oxygen.
FIG. 5A is an exemplary plot of measured data points with a curve calculated to fit the measured data points.
FIG. 5B is another view of the plot of FIG. 5A.
FIG. 5C is another view of the plot of FIG. 5A.
FIG. 5D is another view of the plot of FIG. 5A.
FIG. 6 is an exemplary flow chart of steps to determine patient volume of gas inhaled.
FIG. 7 is another exemplary flow chart of steps to determine patient volume of gas inhaled.
FIG. 8 is a perspective view of an exemplary housing and connector that can be used in the present system.
FIG. 9 is an exemplary flow chart of steps to determine patient tidal volume of gas inhaled.
FIG. 10 is an exemplary graph of flow generator input of pure oxygen and flow generator output of oxygen blended with ambient air over a breath.
FIG. 11 is the exemplary graph of FIG. 10 showing how leak flow may be determined during the end expiratory pause of the breath and subtracted from other phases of a breath to correct for leak flow.
FIG. 12 is the exemplary graph of FIG. 10 showing how a percentage of oxygen can be calculated for the inspiration phase of a breath.
FIG. 13 is the exemplary graph of FIG. 10 showing how a percentage of oxygen can be calculated for the lung re-pressurization phase of a breath.

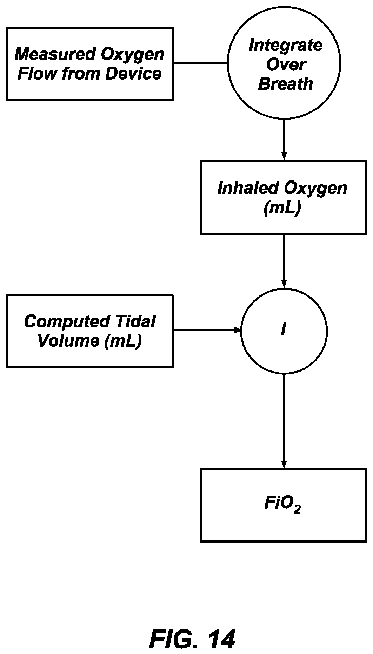

FIG. 14 is a block diagram of steps to determine a fraction of inspired oxygen.

### DETAILED DESCRIPTION

The present disclosure relates generally to a system and device for improved oxygen delivery to a patient as well as a method of measuring volume of gas inhaled by the patient. As used herein, "proximal" refers to a portion of the system or device that is closer to the patient when in use, and "distal" refers to a portion of the system or device that is farther from the patient when in use. For example, the system described herein may be used in conjunction with a patient's mask and the proximal end of the mask is the end closer to the patient and the distal end is the end farther from the patient. The system described herein may include a CPAP adapter or flow generator, and as used herein, "flow generator" means any type of fluid flow control apparatus, such as an adapter, a flow generator, a Venturi adapter, or any other device that can be connected to a patient interface (such as a mask) to input a high pressure oxygen flow into the adapter and output from the adapter a low pressure of oxygen and/or a low pressure of oxygen blended with ambient air. The patient interface could be a mask, an endotracheal tube, a supraglottic airway device (a laryngeal mask airway is common), etc.

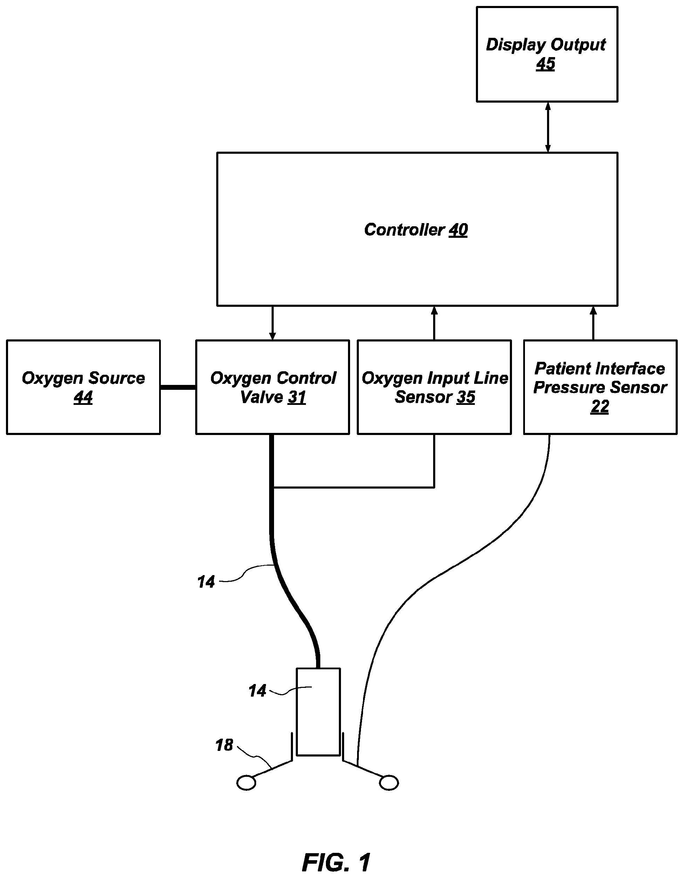

Referring to FIG. 1, an exemplary system is generally indicated at 10. The system 10 generally includes a flow generator 14 connected to a patient interface 18 (patient interface 18 in the exemplary configuration of FIG. 1 is a mask), a patient interface pressure sensor 22, an oxygen input line 27 with an oxygen control valve 31 and an oxygen input line sensor 35, and a controller 40 in communication with the patient interface pressure sensor 22 and the oxygen input line sensor 35. The oxygen control valve 31 controls the flow from an oxygen source 44, through the oxygen input line 27 and to the flow generator 14. An optional output device, such as a display output 45, can be in communication with the controller 40.

Flow generator 14 can be any suitable flow generator known in the art. Flow generator 14 can have known characteristics that are pre-determined or pre-measured. For example, a flow generator with a particular nozzle configuration and dimensions, can have a known or pre-determined oxygen flow output at various oxygen input line pressures and patient interface pressures as described in more detail below. The flow generator 14 takes an input of low-flow, high-pressure compressed oxygen and outputs a high-flow, low-pressure gas source suitable to provide CPAP therapy even in the presence of mask leak.

Flow generators 14 generally use a high velocity jet of oxygen inside of the body of the flow generator to draw in ambient air, thereby creating a higher flow oxygen/air gas blend. The high velocity jet of gas is created by passing high-pressure oxygen through a small-orifice nozzle in the adapter. Because air and oxygen are viscous gases, the jet of oxygen that exits through the small-orifice nozzle pulls ambient air into the flow generator 14 through ports such that when flow is unobstructed, the total flow at the outlet (on the proximal mask/patient side) of the flow generator 14 is much greater than the flow of oxygen input through the small-orifice nozzle. Efficient flow generators 14 generate an output flow that is many times greater than the input flow of oxygen through the small-orifice nozzle, where efficiency is calculated as the quotient of output flow over input flow. The efficiency of the flow generator can be raised by, among other things, increasing the velocity of oxygen as it exits the small-orifice nozzle. Several variations of nozzles are possible, and U.S. patent application no. 17/737,829 describes some exemplary flow generators 14 which can be used in system described herein. Other flow generators known in the art can also be used.

In some configurations the flow generator 14 includes one or more unique identification markers. This unique identification marker can include details relating to the flow generator 14, such as the dimensions of the flow generator and the configuration of the nozzle(s). The controller 40 may be able to automatically determine what type of flow generator 14 is being used by reading or receiving the data from the unique identification marker. For example, near-field communication, RFID, Bluetooth, or other wireless protocols can communicate the data associated with the unique identification marker of the flow generator 14 to the controller 40. Or wired communications can also be used. In other configurations described below, the unique identification marker is included on a connector which is connected to a housing that houses the controller 40.

The flow generator 14 is used to generate a flow into the patient interface 18. Patient interface 18 can include any suitable interface. Masks that cover the nose and mouth are common, and endotracheal tubes, supraglottic airway devices, etc., are also possible. In configurations where the patient interface 18 is an endotracheal tube, the system can include software for controlling the oxygen valve to turn the oxygen flow on and off for a certain duration and at a certain frequency for moving gas in and out of the lungs.

While in use, the patient interface 18 has a pressure which is caused by several different factors. For example, as the patient breathes in, it causes a negative pressure in the patient interface 18. Similarly, the pressure exerted on the patient interface 18 by the flow generator 14 causes a negative pressure. As the patient breaths out, it causes a positive pressure in the patient interface 18.

The pressure in the patient interface 18 is measured by a patient interface pressure sensor 22. The patient interface pressure sensor 22 can be located on the patient interface 18, or the patient interface pressure sensor 22 can be in fluid communication with the patient interface 18. The patient interface pressure sensor 22 can be in communication with one or more controllers and/or processor(s) 40. The controllers and/or processor(s) 40 can receive data, either directly or indirectly, from the patient interface pressure sensor 22. The communication can be either wired or wireless depending on the desired configuration.

Oxygen from oxygen source 44 is delivered to the patient interface 18 through oxygen input line 27. In the exemplary configuration shown in FIG. 1, flow generator 14 receives oxygen through an oxygen input line 27, and the flow generator 14 is connected to the patient interface 18. Oxygen input line 27 is in fluid communication with an oxygen source 44, such as a source of compressed oxygen. Oxygen input line optionally includes an oxygen control valve 31 between the oxygen source 44 and the flow generator. Oxygen control valve 31 can be selectively controlled to change the degree to which the valve 31 is opened and therefore change the flow of oxygen through the oxygen input line 27 to the flow generator 14. Oxygen control valve can be any suitable known valve, and in some configurations, an electronic oxygen control valve can be used and directly controlled by controller 40.

Oxygen control valve 31 can be in communication with controller 40 to control the oxygen control valve 31. The controller 40 may be used to control the oxygen control valve 31 to deliver a specific flow rate of oxygen to a patient. For example, the controller may set a flow rate that changes up to 100 times per second according to variables such as the desired oxygen delivery for the particular patient. The variable-type of flow control valve 31 may be, for example, those known in the art such as the EVP Series Proportional Control Valves manufactured by Clippard Instrument Laboratory, Inc. In other configurations, a separate oxygen control valve 31 may not be provided.

The oxygen flow from the oxygen source 44, when connected to the flow generator 14, converts high pressure oxygen (which is typically around 55 pounds per square inch) into a low pressure oxygen/air blend suitable for CPAP therapy (typically less than 0.35 pounds per square inch or 25 cm H₂O). The flow generator 14 is capable of supplying gas to the patient at high flow rates so that it can maintain patient interface pressure even when the patient is inhaling or when the patient interface 18 leaks. The patient interface pressure sensor 22 can be connected to the patient interface 18 so that controller(s) 40 can receive the patient interface pressure and automatically adjust oxygen flow by adjusting oxygen control valve 31 to maintain the patient interface pressure at the prescribed CPAP pressure.

In the exemplary configuration shown in FIG. 1, oxygen input line 27 has an oxygen input line sensor 35 in fluid communication with the oxygen input line 27. Oxygen input line sensor 35 is used to measure a flow within the oxygen input line 27, before the flow generator 14. In configurations with an oxygen control valve 31, the oxygen input line sensor 35 can be downstream of the oxygen control valve 31. The oxygen input line sensor 35 can also be in communication with one or more controller(s) 40. The controller(s) 40 can receive data, either directly or indirectly, from the oxygen input line sensor 35. The communication can be either wired or wireless depending on the desired configuration.

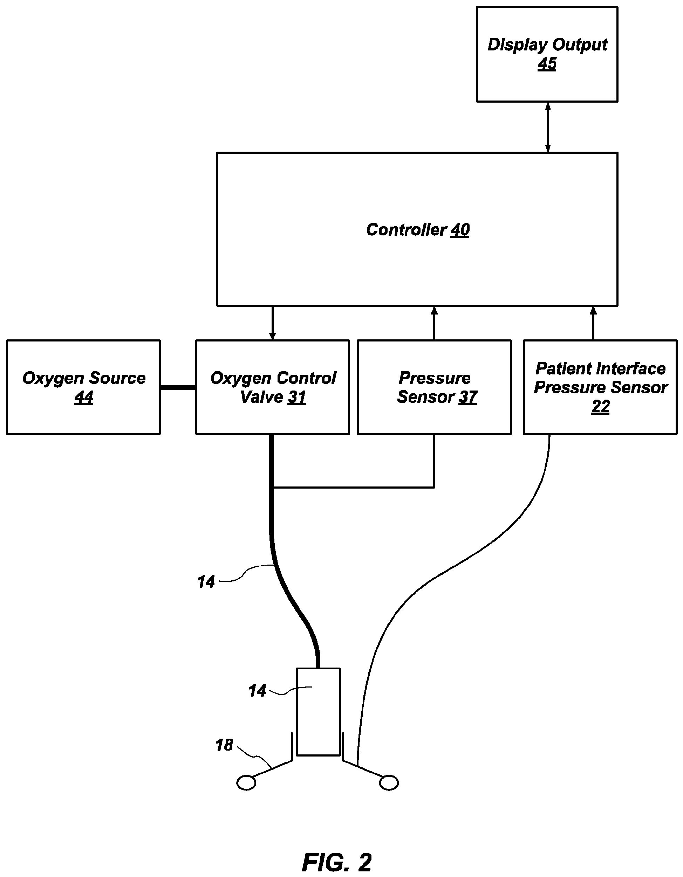

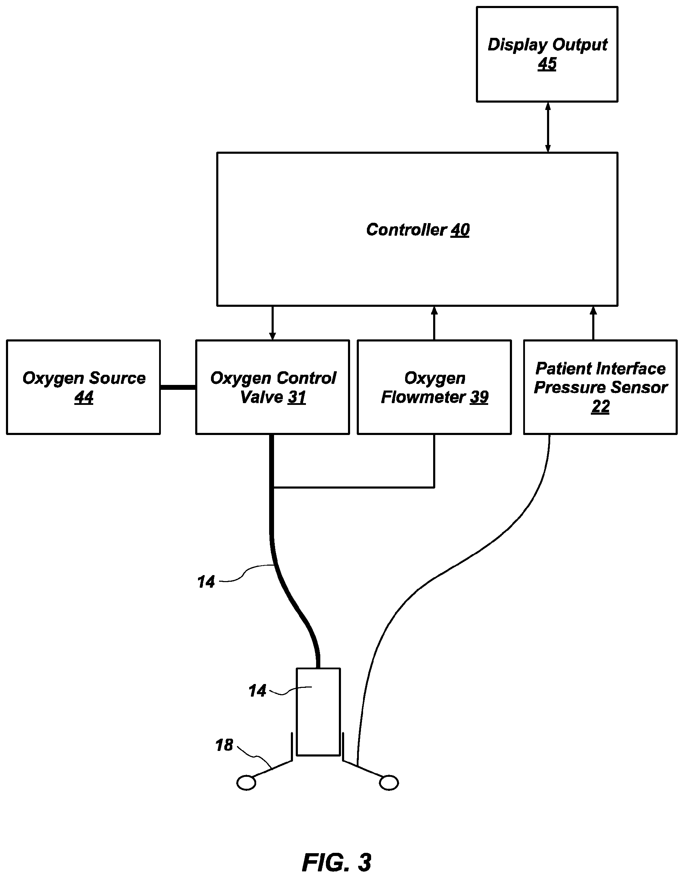

In some configurations, an oxygen input line sensor 35 comprises a pressure sensor 37 (FIG. 2). In other configurations, a pressure sensor is not included. Rather, oxygen input line sensor 35 includes an oxygen flowmeter 39 to directly measure oxygen flow (for example, differential pressure, turbine, heated wire, or other oxygen flow measurement technology known in the art, see FIG. 3). Where the characteristics of the oxygen input line 27 and flow generator 14 are known, the pressure in the oxygen input line 27 can be used as a substitute for the flow measurement.

Because the pressure in the oxygen input line 27 is much higher than the pressure in the patient interface 18 and because the resistance to gas flow through the nozzle of the flow generator has been characterized, the oxygen flow rate can be effectively calculated by using a pressure sensor as the oxygen input line sensor 35. The oxygen input line pressure as measured by the pressure sensor 37 can be substituted for the oxygen flow measurement or can be considered as an oxygen flow measurement. The measured oxygen flow signal can either be taken as the directly measured oxygen flow rate as measured by an oxygen flowmeter 39 (FIG. 3), or the directly measure oxygen input line pressure as measured by a pressure sensor 37 (FIG. 2), or any other directly measured signal that is proportion to the flow rate of oxygen into the flow generator 14.

In the exemplary configuration shown in FIG. 1, the controller(s) 40 include a microcontroller or controller with memory, processor, and input/output peripherals. The controller 40 can be programmed to receive data relating to the pressure at the patient interface 18 from the patient interface pressure sensor 22, data relating to the pressure at the oxygen input line 27 from the oxygen input line sensor 35, and use this data to determine an oxygen flow output from the flow generator 14. The processor can also be programmed as a CPAP device to receive the patient interface pressure and automatically adjust oxygen flow through oxygen input line 27 by adjusting oxygen control valve 31 to maintain the patient interface pressure at the prescribed CPAP pressure.

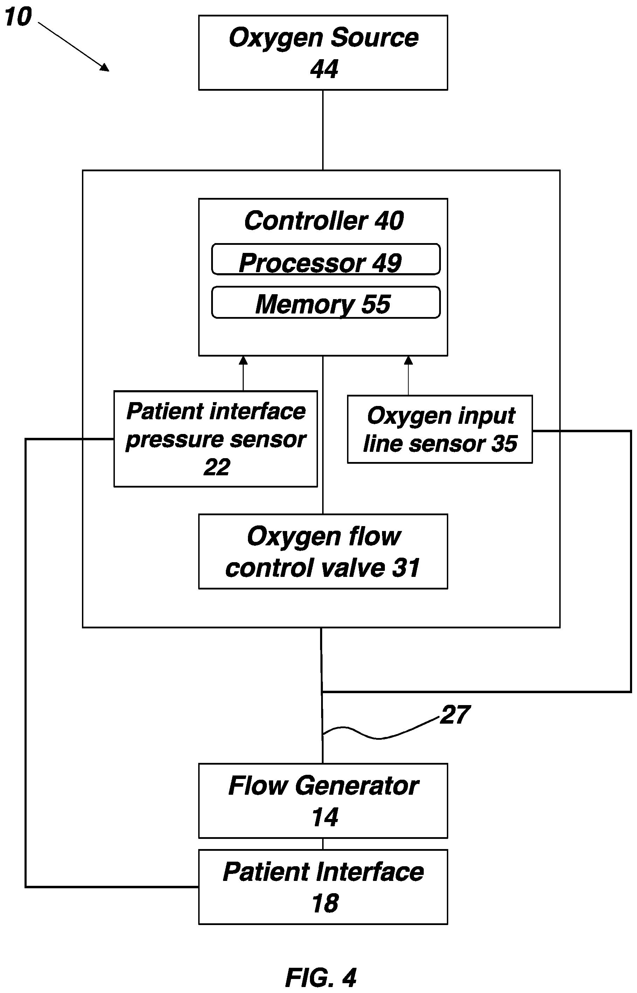

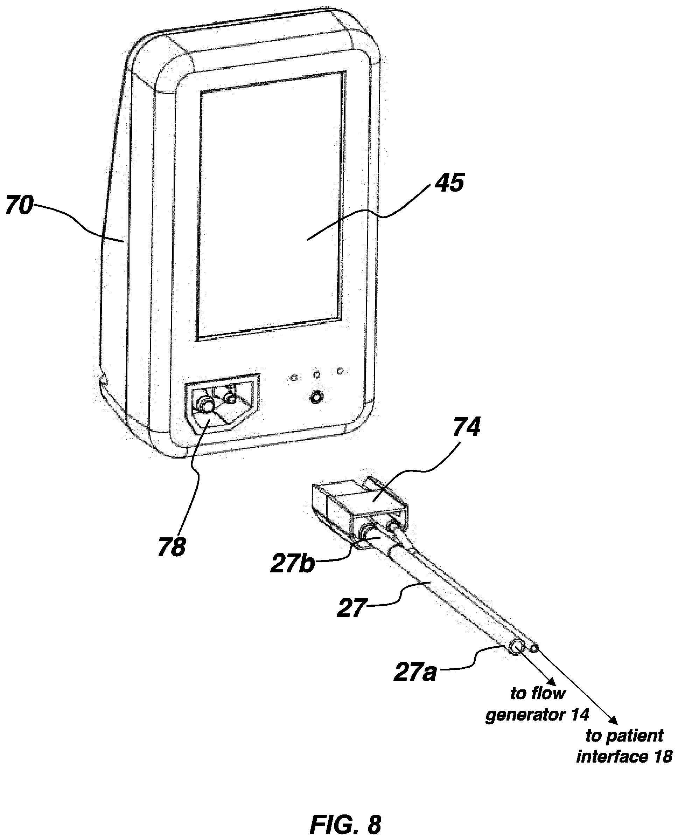

An exemplary configuration of a controller 40 which can be used in an oxygen delivery and/or measurement system is shown in the block diagram of FIG. 4. The exemplary controller 40 can include one or more processors 49, memory 55, and programmable input/output peripherals (such as a display output 45, see FIG. 8), and the disclosed embodiments are not limited to any specific type of controller(s) or processor(s). The controller 40 may be in communication with memory 55, which may include one or more storage devices configured to store instructions used by the processor 49 to perform functions related to disclosed embodiments (such as closing and/or opening the oxygen control valve 31, calculating a flow generator flow output, etc.). For example, the memory 55 may be configured with one or more software instructions, such as programs that may perform one or more operations when executed by the processor 49. The disclosed embodiments are not limited to separate programs or computers configured to perform dedicated tasks. For example, memory 55 may include a single program that performs the functions of the processor 49, or memory 55 may comprise multiple programs. Memory 55 may also store data that is used by one or more programs (such as look-up tables, algorithms, characteristics of unique types of flow generators, pre-set oxygen delivery flow rates, predetermined time intervals, predetermined time periods, etc.), and/or a controller 40. In some configurations, memory 55 and associated applications may be stored within the system 10, and in other configurations, memory 55 and associated applications may be stored at a remote location for storage and processing by the controller 40 and/or in the cloud, such as a network, etc.

The functions of the various elements shown in the figures, including any functional blocks labeled as "controller(s)" and/or "processor(s)", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), flash memory, and/or nonvolatile storage. Other hardware, conventional and/or custom, may also be included within the controller 40 and/or oxygen delivery system 10.

In some configurations, the controller 40 may be programmed, through one or more processor(s) 49 and memory 55, to determine the oxygen flow output from the flow generator 14. The controller 40 can determine total flow output from the flow generator 14 in several different ways. The flow generator 14 includes air input ports that are open to ambient air such that there is no direct measurement of air flow into or out of the flow generator. Additionally, when the flow exiting the flow generator 14 at its exit port is blocked (such as when a patient breathes out), oxygen flow entering the flow generator 14 from the oxygen input line 27 can escape from the flow generator 14 through the air input port(s) of the flow generator 14 rather than through the exit port of the flow generator into the patient mask. So instead of directly measuring the output flow of the flow generator 14 and the input flow of the flow generator 14, patient interface pressure and oxygen input line pressure sensor can be measured directly and provided to the controller 40, and an algorithm can be applied to calculate the output flow of the flow generator 14 for each possible combination of output pressure and oxygen flow.

As discussed above, characteristics and parameters of a particular flow generator 14 can be known or predetermined. For example, for a given flow generator 14 having predetermined dimensions and a predetermined configuration of nozzle(s), the output flow can be known by prior measurement for a given patient interface pressure and an oxygen input line pressure sensor. CPAP flow generators are designed to increase volume of inhaled oxygen, and the concentration of oxygen in the gas exiting the flow generator (i.e., the flow generator flow output) changes as a function of the oxygen input line flow rate and the patient interface pressure. By mapping this function of measured flow output at various known oxygen input line flow rates and patient interface pressure, an algorithm unique to a particular flow generator can be created.

Algorithm parameters are unique for each flow generator design and dimensions. In other words, algorithm parameters are specific for the unique design of a particular type of flow generator. The algorithm can be as simple as a pre-stored lookup table, a polynomial curve fit or other method of reporting a pre-determined output value for a given combination of mask (output) pressure and oxygen flow rate. The parameters of the algorithm are predetermined. This can typically be done on the test bench by generating multiple combinations of oxygen input line 27 flow and patient interface pressure and measuring corresponding output flow using calibrated equipment. Flow generator characterization data can also be collected while directly controlling the patient interface pressure or while applying a flow restrictor, or a range of flow restrictions, to the output of the flow generator and recording the output flow corresponding to applied oxygen flow rates.

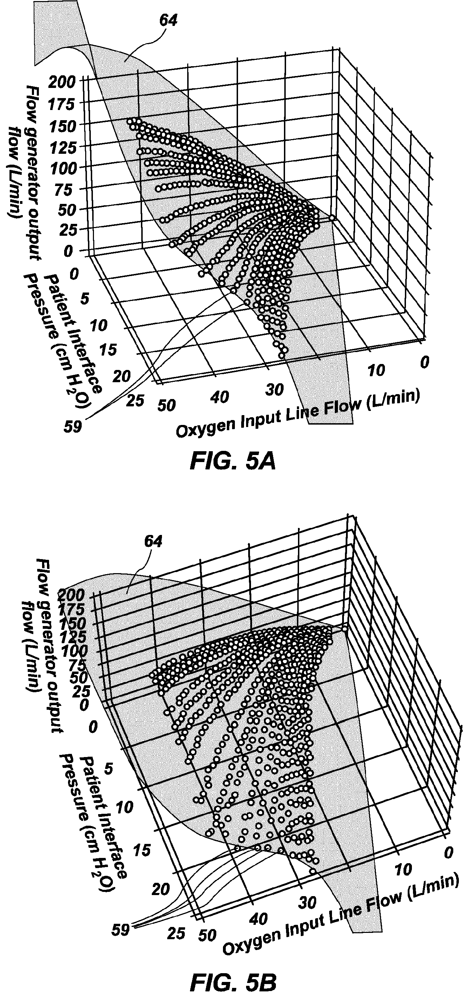

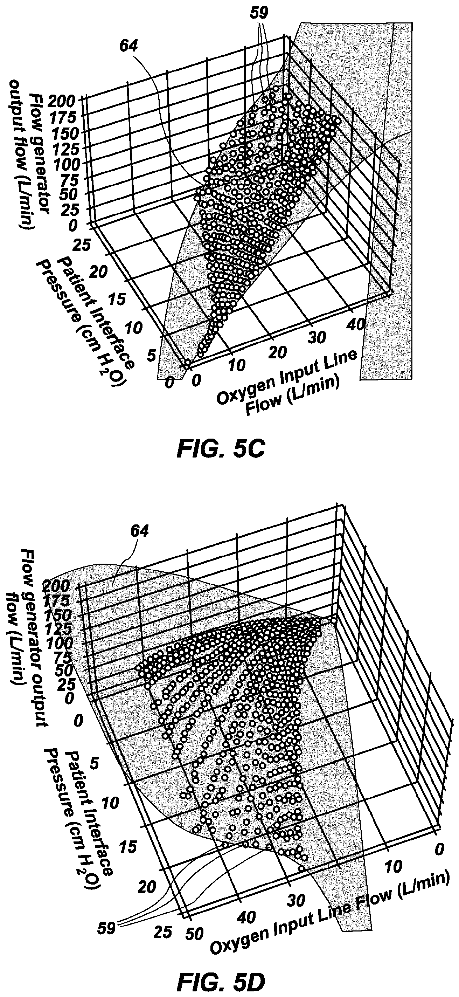

FIGs. 5A-D show various views of a single exemplary plot of data, with known data points shown as circular points 59. The data show the measured flow generator output collected at multiple combinations of patient interface pressures measured by the patient interface pressure sensor 22 and input oxygen flow rates, or oxygen tube pressure. These exemplary data points 59 were taken by measuring oxygen input line 27 flow (in L/min) as measured by the oxygen input line sensor 35, patient interface pressure (in cm H₂O) as measured by the patient interface pressure sensor 22, and the measured flow output of the flow generator 14 which is measured at the test bench using a gas flow analyzer. As mentioned above, the oxygen flow rate can either be measured directly by an oxygen flow meter or taken from an oxygen line pressure sensor.

After enough data points are collected, they can be input into a controller 40. For example the data points can be stored in memory 55, and an algorithm (shown as the curve 64 fit to the data points 59 in FIGs. 5A-D) can be generated. The surface 64 in FIGs. 2A-D shows a polynomial function defined to fit the collected data. This fitted function can be used to calculate adapter output flow for any combination of oxygen flow and patient interface pressure.

By measuring the flow generator 14 output flow for many possible combinations of measured oxygen flow rates through oxygen input line 27 and measured patient interface pressure for a specific flow generator 14, the flow generator 14 can be functionally characterized. This oxygen flow and patient interface characterization data can be used to generate data tables and/or algorithm parameters that are used to determine what output flow to report for a given combination of oxygen flow and patient interface pressure. This allows the output flow rate for a particular flow generator 14 to be determined for any given combination of input oxygen flow and measured patient interface pressure.

An algorithm and pre-collected data or algorithm parameters can be applied which use directly measured oxygen flow and patient interface pressure as inputs to determine the concentration of oxygen in the gas exiting the flow generator (i.e., the patient flow). Any suitable algorithm can be used to convert a known patient interface pressure and oxygen input line flow to an estimated flow generator output. By way of example and not limitation, this can be done by using a pre-defined mathematical function, a polynomial, or other, curve fit, a lookup table, an artificial neural network trained using the characterization data, a nearest neighbor search algorithm or other numerical method used to define an output value given two or more input values.

While the description above refers to determining the output flow of the flow generator 14, flow can also be calculated as pressure divided by resistance. By determining the resistance observed at the patient interface 18, the flow becomes trivial. Therefore, instead of calculating the flow output of the flow generator 14, the output resistance of the flow generator 14 can be calculated.

With a knowledge of the output flow of the flow generator 14, the flow into the patient interface 18 can be integrated over the breath to calculate the patient's inspiratory tidal volume or the volume inhaled during each single breath. Breath volume in turn can be used to determine the volume of gas inhaled by the patient each minute. This parameter is often used by clinicians to determine patient status. Without this method of determining the output flow from the flow generator 14, a direct flow measurement device between the patient interface 18 and the flow generator 14 would be needed. This could be costly and can also interfere with patient care if the direct flow measurement device is not properly positioned within the system.

In some configurations, the system further comprises a method of determining the tidal volume for the patient. Tidal volume is the amount of air that moves in or out of the lungs with each respiratory cycle. The inspired breath volume, or tidal volume, is calculated as the sum (or integral of) flow over the course of the inspiratory phase of the breath with leak volume subtracted over this same phase. To determine tidal volume, the method can include the step of calculating or determining patient interface 18 leak. When there is leak around the patient interface (such as leak around a mask seal), all of the flow exiting the flow generator 14 is not inhaled by the patient. That is, some of the flow output by the flow generator 14 is lost to leakage.

By subtracting the amount of leakage from the total output flow of the flow generator 14, the method can more accurately determine the output flow that is delivered or inhaled by the patient. To determine the leak rate, the method includes the step of determining the flow rate from the flow generator during the period of the end-expiratory pause at the end of each exhalation. During this pause at the end of exhalation, the patient is resting and there is no movement of gas into or out of the patient. Because there is no movement of gas into or out of the patient, during this time all output flow from the flow generator 14 is attributed to leak flow. This leak flow rate can be measured breath by breath, and on the subsequent breath(s) that leak flow is subtracted from the total flow generator output flow to calculate the flow that is inhaled by the patient during that particular breath. This sum of the flow inhaled by the patient during the breath can be used to determine tidal volume.

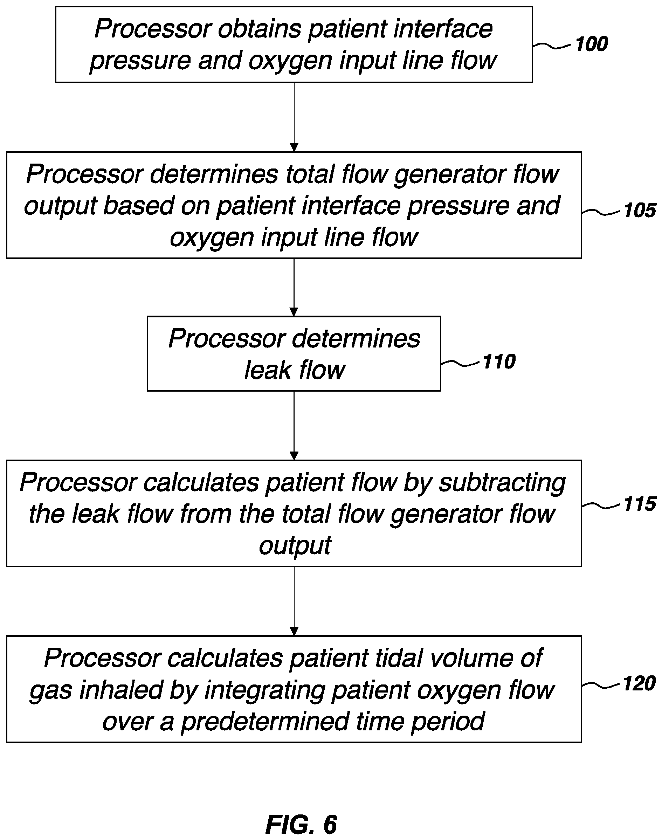

With a calculated mask leak rate, which is subtracted from the determined total flow generator flow output, a volume of gas inhaled by a patient can be calculated. FIGs. 6-7 show exemplary configurations of a process that can be used to calculate patient tidal volume of oxygen inhaled. While the steps are shown as discrete steps occurring in an order for clarity, most of the steps shown in FIG. 6 will be performed (and the associated calculations updated), continuously or nearly continuously. For example, the patient interface pressure and the oxygen input line flow can be measured and reported to the controller many times per second.

The method can include the step 100 of obtaining, by a processor, information relating to a patient interface pressure and an oxygen input line flow. This includes the patient interface pressure, which can be measured, for example, by patient interface pressure sensor 22. This also includes the oxygen input line flow, which can be measured, for example, by a flowmeter and/or an oxygen input line pressure sensor. The processor then determines (step 105) a total flow generator flow output based on the patient interface pressure and the oxygen input line flow. This can be done in any suitable manner, such as through an algorithm, lookup tables, etc.

After the total flow generator flow output is determined, the processor can then determine a leak flow (step 110). Leak flow is determined by first identifying the period of the end-expiratory pause at the end of an exhalation. This can be done, for example, by the processor/controller 40 tracking a breath over time by measuring the patient interface pressure over time to determine when there is a pause in breathing. The pause is detected as a period of unchanging mask pressure and oxygen flow. Inhalation is detected as the period when added oxygen flow is needed to maintain mask pressure. Exhalation is the period when less oxygen flow into the flow generator is needed to maintain set mask pressure. The pause is the time between these two conditions.

At the pause before the patient begins their next inhalation (in which there is no movement of gas into or out of the patient), the processor can measure the patient interface pressure and the oxygen input line flow and determine the output flow from the flow generator 14. At this pause, all output flow from the flow generator 14 is attributed to leak flow.

After the processor determines leak flow, the processor can calculate the patient flow (step 115) by subtracting the leak flow from the total flow generator flow output. Once the patient flow is known, the processor can calculate the patient tidal volume of gas inhaled by integrating the patient flow over a predetermined time period. For example, the patient tidal volume of gas for a single breath can be determined by integrating the patient flow over the time period of an inhalation. In some examples, the patient tidal volume of gas inhaled for a given time period (for example, for one minute) can be determined by integrating the patient flow for each of the inhalations during that time period (step 120).

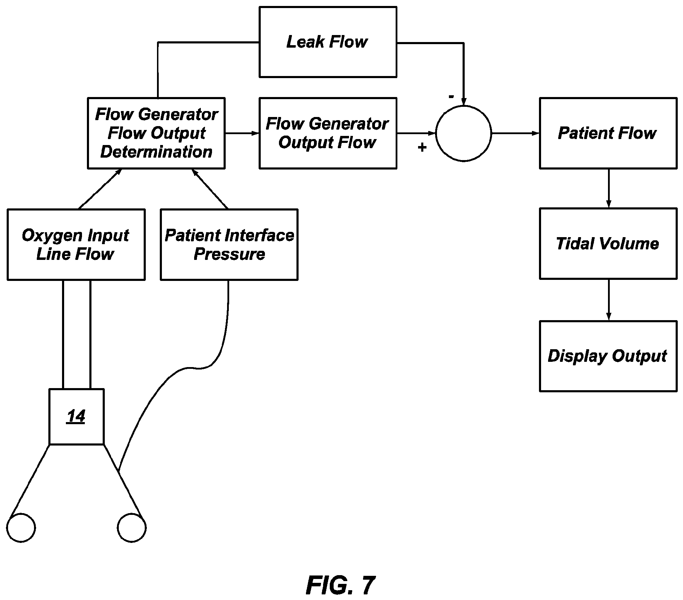

In addition to determining the patient tidal volume of oxygen inhaled, the controller 40 can further output these details to a display (see FIG. 7). The display and/or associated input/output device can be programmed to include one or more visual and/or audible alerts for clinicians. In some configurations, the display can automatically include a visualization showing a range of tidal volume that is typical and acceptable for a patient. Where the patient's tidal volume falls outside this range, a visual alert can appear on the display 45, and/or an audible alarm can be activated to alert clinician(s) to the patient's status.

Another feature of the system 10 can include the ability of the controller 40 to automatically determine what type of flow generator 14 is being used. The controller 40 can have memory 55 that is programmed with algorithms and look-up tables for several different types of flow generators. The controller 40 can automatically determine what type of flow generator 14 is being used and apply the appropriate algorithm for that particular flow generator 14. This can be accomplished in several ways. For example, the flow generator 14 can have a unique identifier located on the flow generator (such as a QR code, an alpha-numeric code, etc.). In some configurations, the clinician inputs this unique code into the system to inform the controller. In other configurations, the controller 40 detects this unique code automatically through the use of wired or wireless technology.

An exemplary configuration of a housing 70 to house controller 40 and other input/output devices is shown in FIG. 8. In this configuration, housing 70 can be fluidly connected to an oxygen source 44, and housing 70 houses the controller 40, as well as an oxygen control valve 31, an oxygen input line sensor 35, and/or a patient interface pressure sensor 22. In other configurations, not all these components are housed in a single housing.

A connector 74 is inserted into a receptacle 78 on the housing 70. Connector 74 can have a unique, readable code or type-identifier thereon and receptacle 78 can have a reader to read the code. Many configurations are possible to achieve this result, and several are discussed in U.S. Patent No. 10,737,049. By way of example and not limitation, one or more of the following can be used: a code printed on the connector 74, a gradient printed on the connector 74, an angled cut-away formed in the connector 74, and/or a reflective pad on the connector 74.

Connector 74 includes an oxygen input line 27 which is connected to flow generator 14. The distal end 27b of the oxygen input line is attached to the connector 74. In some configurations, the distal end 27b of the oxygen input line 27 is non-removably attached to the connector 74. The proximal end 27a of the oxygen input line is attached to a flow generator 14. The unique, readable code on the connector 74 is specific to the type of flow generator 14 which is connected to the connector 74. Different flow generators have different readable codes on their specific connectors 74. This readable code is read by one or more input/output devices in communication with the controller 40. The controller 40, in turn, applies the algorithm that is appropriate for the unique flow generator that is detected.

In yet another example, the current system can also be used to calculate the fraction of inspired oxygen (FiO₂). FiO₂ is the percent of the percent of the total volume of gas entering the patient's lung that is comprised of oxygen. Since the total gas volume is a mix of oxygen and entrained ambient air, it can be useful to clinicians to know how much of the patient's breath is oxygen. Determining the FiO₂ can be accomplished according to different methods.

The system described above measures the flow of gas that exits the flow generator and the portion of gas that is inhaled by the patient. Because the flow generator uses compressed oxygen as its input gas, the gas entering the mask has a higher fraction of oxygen than ambient room air. The concentration of oxygen in the gas exiting the flow generator is a function of the oxygen flow and the mask pressure. In the same way that the flow generator can be characterized to measure the total gas flow at its output, the flow generator can also be characterized to measure the concentration of oxygen in the gas that exits the flow generator. This is done by measuring the oxygen concentration at the output of the flow generator while creating all possible combinations of oxygen flow and mask pressure. These measurements are then used to calculate oxygen fraction (concentration) in the gas exiting the flow generator using a method similar to that described above.

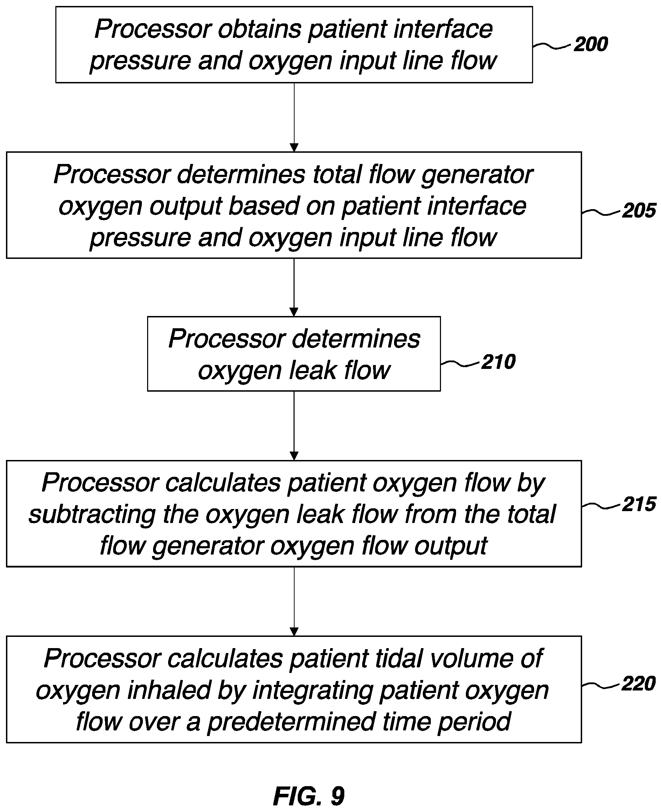

FIG. 9 outlines an exemplary method for determining tidal volume of oxygen inhaled by a patient. The method can include the step 200 of obtaining, by a processor, information relating to a patient interface pressure and an oxygen input line flow. This includes the patient interface pressure, which can be measured, for example, by patient interface pressure sensor 22. This also includes the oxygen input line flow, which can be measured, for example, by a flowmeter and/or an oxygen input line pressure sensor. The processor then determines (step 205) a total flow generator oxygen flow output based on the patient interface pressure and the oxygen input line flow. This can be done in any suitable manner, such as through an algorithm, lookup tables, etc.

After the total flow generator oxygen flow output is determined, the processor can then determine an oxygen leak flow (step 210). Oxygen leak flow is determined by first identifying the period of the end-expiratory pause at the end of an exhalation. This can be done, for example, by the processor/controller 40 tracking a breath over time by measuring the patient interface pressure over time to determine when there is a pause in breathing. The pause is detected as a period of unchanging mask pressure and oxygen flow. Inhalation is detected as the period when added oxygen flow is needed to maintain mask pressure. Exhalation is the period when less oxygen flow into the flow generator is needed to maintain set mask pressure. The pause is the time between these two conditions. At this pause, all output flow from the flow generator 14 is attributed to leak flow.

After the processor determines oxygen leak flow, the processor can calculate the patient oxygen flow (step 215) by subtracting the oxygen leak flow from the total flow generator oxygen flow output. Once the patient oxygen flow is known, the processor can calculate the patient tidal volume of oxygen inhaled by integrating the patient oxygen flow over a predetermined time period. For example, the patient tidal volume of oxygen for a single breath can be determined by integrating the patient flow over the time period of an inhalation. And the total patient tidal volume of gas inhaled for a given time period (for example, for the last breath, for the last one minute, etc.) can be determined by integrating the patient flow for each of the inhalations during that time period (step 220).

Another method of determining FiO₂ is determining the percentage of oxygen in the total volume of gas output by the flow generator. The percentage of oxygen can be determined by computing the volume of pure oxygen included in the total volume inhaled by the patient and dividing by the total volume of gas inhaled. To determine the volume of pure oxygen inhaled, two flow measurements are considered: the input flow into the flow generator (which is pure oxygen from oxygen source 44) and the output flow from the flow generator (which is a mix of the pure oxygen from the oxygen source 44 and entrained ambient air which is 21% oxygen).

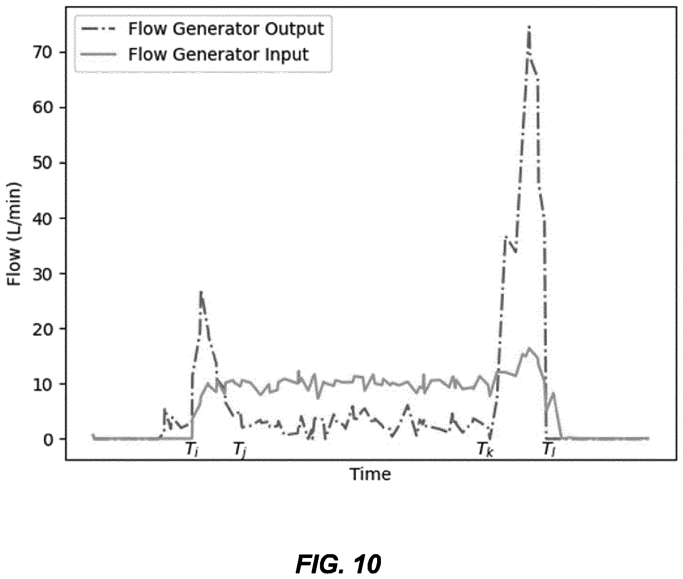

FIG. 10 shows a graph of a waveform of the input flow and the output flow for a sample breath during an exemplary use of the flow generator for a patient having CPAP therapy. The x-axis shows time and indicates four different phases of an exemplary breath. The four phases are lung re-pressurization, end-expiratory pause (EEP), inhalation, and exhalation. Time axis marker Tᵢ indicates the end of exhalation and the start of lung re-pressurization. Tⱼ indicates the end of lung re-pressurization and the start of EEP. Tₖ indicates the end of EEP and the start of inhalation. Tⱼ indicates the end of inhalation and the start of exhalation. Gas flow in and out of the patient occurs during all phases of the breath except for EEP (although EEP still impacts inhaled oxygen, as discussed below).

During exhalation, some amount of gas is pushed out of the patient's lungs. After the patient is done exhaling, lung re-pressurization occurs, and the flow generator pushes some amount of gas into the lungs in order to raise the patient's airway pressure back up to the set CPAP pressure. Thus some amount of gas (mix of oxygen and ambient air) enters the patient even before inhalation. Then during EEP there is no gas movement in or out of the patient until inhalation, when a larger amount of oxygen and ambient air mixture is brought into the patient's lungs.

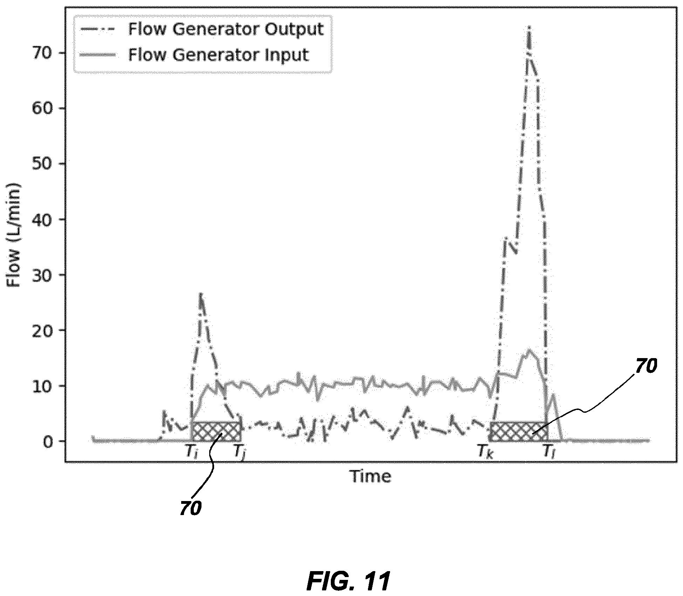

As discussed above, leak flow can be determined by the ouput of the flow generator during EEP (see FIG. 6, step 110 and FIG. 7). FIG. 11 shows a chart illustrating how leak flow is determined. The flow generator flow output is measured during EEP (between time Tⱼ and Tₖ marked on the x-axis), and then this amount is subtracted from the oxygen delivered during other phases of the breath. As seen in FIG. 11, the average flow (in L/min) during EEP is subtracted from oxygen delivered during other breath phases. This subtracted amount is indicated as 70 in FIG. 11 during lung re-pressurization (from Tᵢ to Tⱼ) and during inhalation (from Tₖ to Tₗ).

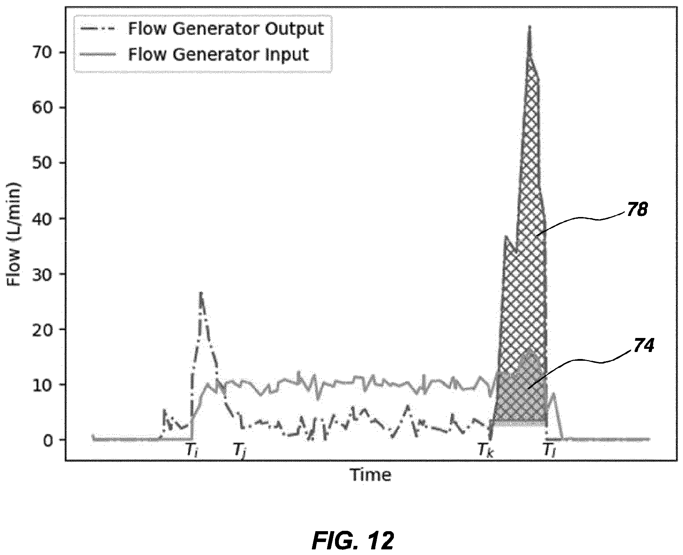

The volume of gas and oxygen output by the flow generator during inhalation (from Tₖ to Tₗ) is computed by integrating the input flow, see FIG. 12. Leak flow is subtracted out. The amount of total output flow that is greater than the input flow (indicated at 78) is assumed to be from entrained air and assigned an assumed volume of 21% oxygen. The amount of total output flow that is due to the input flow (indicated at 74) is assumed to be from the input flow of 100% oxygen. The concentration of oxygen is determined by dividing the total sum of the oxygen flow during the time period (the input flow and 21% of the difference between the output flow and the input flow) by the total output volume over the time (and subtracting leak flows).

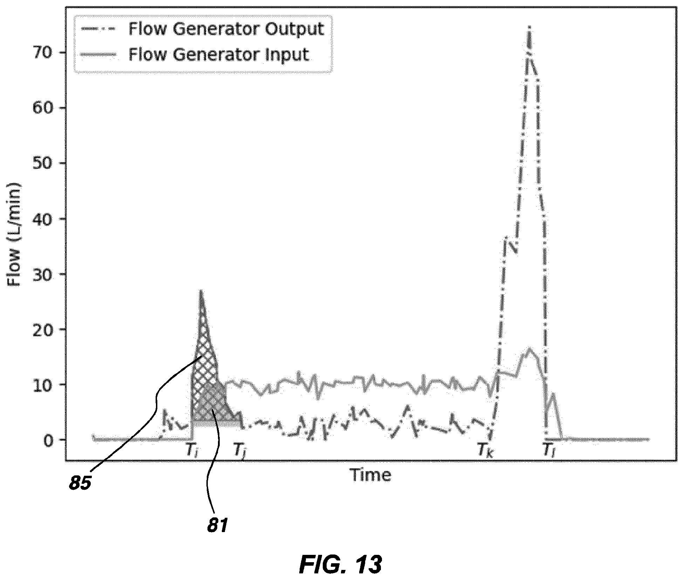

In some configurations, the amount of oxygen delivered to a patient during lung re-pressurization can also be calculated. FIG. 13 shows an illustration of this method, which is similar to the method described above for determining the amount of oxygen delivered during inhalation. Lung re-pressurization begins at Tᵢ and ends at the beginning of EEP, indicated at Tⱼ.

After the patient is done exhaling, lung re-pressurization occurs, and the flow generator pushes some amount of gas into the lungs to raise the patient's airway pressure back up to the set CPAP pressure. Thus some amount of gas (mix of oxygen and ambient air) enters the patient even before inhalation. There is also a certain amount of space in the patient's trachea as well as space in the patient's mask at the patient interface that impacts the FiO₂. The volume of the mask can be approximately measured, but the volume of the patient's trachea is typically estimated by one or more known estimation methods. The combination of these two spaces is referred to as "dead space."

After exhalation, the dead space is filled with exhaled gas that has been expelled from the lungs, which is comprised of some amount of oxygen (typically about 5-6% less than whatever the previous amount of inhaled oxygen was). During lung re-pressurization, this gas in the trachea and mask is the first to get pushed into the lungs followed the gas output by the flow generator that is needed to get the patient's airway up to the set CPAP pressure.

After the gas from the trachea and mask are pushed into the lungs, the trachea and mask space are filled with gas with an oxygen concentration which was output by the flow generator 14 during the lung re-pressurization. This concentration is computed similar to the method of computing the oxygen concentration that was output by the flow generator 14 during inhalation. Both the input flow 81 (assumed to be 100% oxygen) and output flow 85 (assumed to be 21% for the amount that is greater than the input flow, or the difference between the output flow 85 and the input flow 81) are integrated over the duration of lung re-pressurization (with the leak flow volume subtracted from each). The difference between the volumes from output flow 85 and input flow 81 account for the entrained air, which is comprised of approximately 21% oxygen. Adding that 21% to the input volume and dividing it by the output volume from this phase of the breath gives the concentration of oxygen output by the flow generator 14 during lung re-pressurization.

The total amount of oxygen that enters the lungs during the lung re-pressurization phase is the sum of the product of the dead space volume and the previous FiO₂ percentage and the product of the lung re-pressurization oxygen concentration and the difference between the total lung re-pressurization volume and the dead space volume. In other examples, the total amount of oxygen that enters the lungs during the lung re-pressurization phase can be calculated as the sum of the product of the dead space volume and a percentage slightly less than the previous FiO₂ percentage and the product of the lung re-pressurization oxygen concentration and the difference between the total lung re-pressurization volume and the dead space volume. Mathematically this can be represented as: lung_re-pressurization_oxygen_volume = dead_space_volume*previous_FiO2 + lung_re-pressurization_oxygen_concentration*(lung_re-pressurization_total_volume - dead_space volume).

During EEP, no gas is entering the patient, but the flow that is being output by the flow generator pushes out the volume of the mask and replaces it with gas of a new concentration of oxygen. This oxygen concentration is characteristic of the design of the flow generator along with the amount of leak present. This EEP oxygen concentration is a value that can be mathematically characterized for a given adapter design based on the amount of leak flow present. By the end of EEP, at time Tₖ, the trachea volume is filled with gas with the lung re-pressurization oxygen concentration, and the mask is filled with gas with the oxygen concentration that has been characterized from the adapter and leak flow present.

Consideration of the oxygen concentration of the dead space can also be made when calculating the concentration of oxygen inhaled during inhalation. During inhalation, the first gas to enter the patient's lung is the gas in the dead space, and then the volume that is output by the flow generator. Therefore, assuming the volume inhaled by the patient is greater than the volume of the dead space, the total volume of oxygen in the lungs is the sum of the oxygen from the lung re-pressurization, the dead space oxygen, and the concentration of oxygen from inhalation times the total inhaled volume subtract the dead space.

**In** the case where the total volume inhaled is less than the dead space volume but greater than the tracheal volume, the total volume of oxygen inhaled is the sum of the oxygen from lung re-pressurization, the lung re-pressurization oxygen concentration times the tracheal volume, and the characterized EEP concentration times the difference between total inhaled volume and tracheal volume (to account for oxygen in the mask).

In the case where the total volume inhaled is less than the tracheal volume, the total volume of oxygen in the lung is the sum of the oxygen from lung re-pressurization and the lung re-pressurization oxygen concentration times the total inhaled volume.

### Exemplary calculation of FiO₂ over a breath

The following is an exemplary calculation of the fraction of inspired oxygen during an exemplary patient breath. The specific numbers provided are given by way of example and not limitation, and the principals of the calculations set forth below can be applied to other breaths with different numerical data measurements and assumptions. For this exemplary calculation, input flow and output flow have been measured over the time of a breath. The following assumed values are used: previous breath has FiO₂ of 80%; volume of the patient's trachea volume is 150mL; and volume of the mask is 100mL. The dead space volume is the sum of the trachea and mask volumes. These assumptions are shown as:

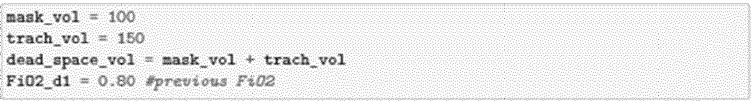

The leak flow is found by examining the flow generator output flow during EEP. Since this may be a noisy signal, averaging or filtering can be applied to obtain the approximately constant leak flow during EEP, which can be used to find the leak volume during lung re-pressurization as well as inhalation. This can be done a number of ways, and in this specific example a recursive median filter is used to reduce noise spikes, and sort the samples into a histogram. The histogram bin with the highest occurrence is used as constant flow value during EEP:

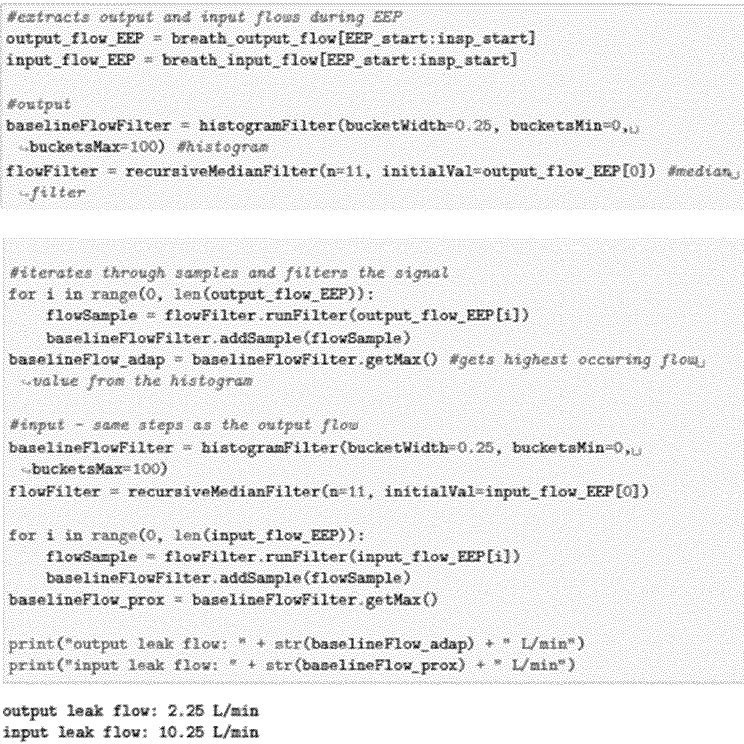

The oxygen concentration of the leak flow is an intrinsic characteristic of the adapter design and the amount of leak flow present. For this particular flow generator with the given leak flow, the oxygen concentration has been determined by characterizing this behavior through sampling the oxygen concentration given different leak flows. This was fit to two linear equations where the input is the ratio of the baseline flow (or leak flow) for the output flow over the baseline flow (or leak flow) for the input flow: 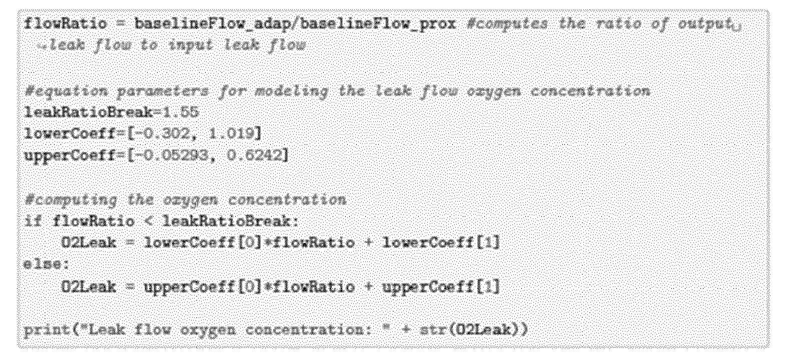 Leak flow oxygen concentration: 0.9527073170731706

To find the volume of oxygen and the volume of total gas during lung re-pressurization that contribute to the patient's FiO₂, both the input and output flows are integrated over the course of the re-pressurization: 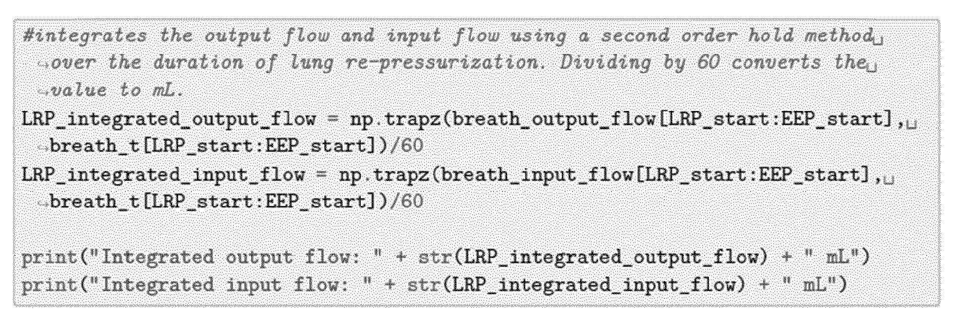
Integrated output flow: 107.7281666666667 mL
Integrated input flow: 67.57558333333333 mL
Output leak flow is integrated over this same duration to get the volume of gas lost to leak during lung re-pressurization: 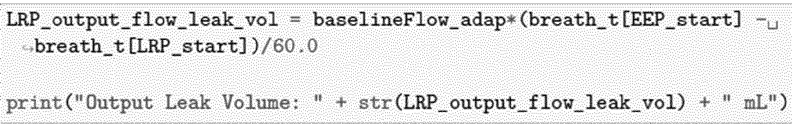 **Output Leak Volume: 31.95 mL**

The amount of this leak volume that is credited to oxygen can be computed by multiplying the oxygen leak concentration by the leak volume. Additionally, to account for the 21% oxygen contained in the entrained air portion, 21% of the difference between the total leak volume and the pure oxygen leak volume can be added to get the total volume of oxygen from leak flow: 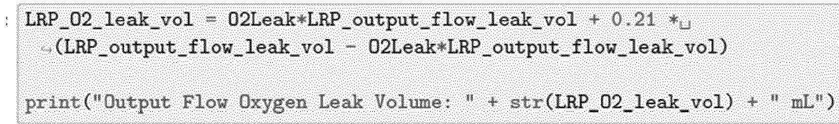 **Output Flow Oxygen Leak Volume: 30.75630903658536 mL**

With the leak volumes for lung re-pressurization known, the total amount of oxygen and total amount of gas that is output by the flow generator that is not lost to leak during this phase of the breath can be computed. Leak flows are subtracted from the integrated values calculated above, and then as done previously, oxygen content in the entrained air is accounted for by adding 21% of the difference to the oxygen volume: 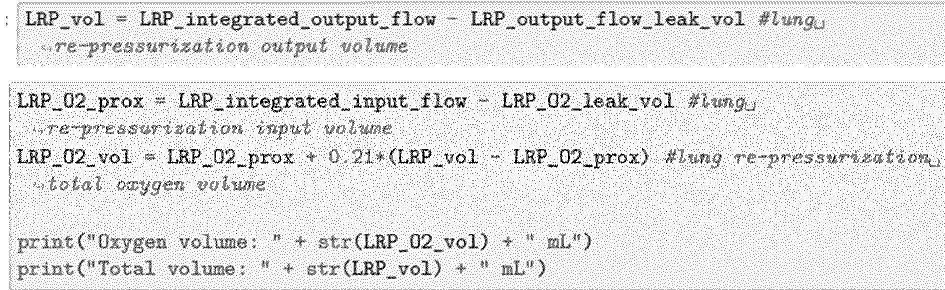
**Oxygen volume: 45.0006416944309 mL**
**Total volume: 75.77816666666669 mL**

Because of the dead space, not all of this volume reaches the patient's lungs at this time. The volume of the dead space (with an oxygen concentration of the previous FiO₂) gets pushed into the lungs first, and the dead space is then left being filled with the concentration of oxygen that is output by the flow generator. However, if the total lung re-pressurization volume is smaller than the dead space, then the entire volume entering the lung is derived from the dead space. This is the case with this exemplary breath, so the volume of gas entering the lung has an oxygen concentration of the previous breath's FiO₂. The total volume of oxygen entering the patient's lung is computed as: 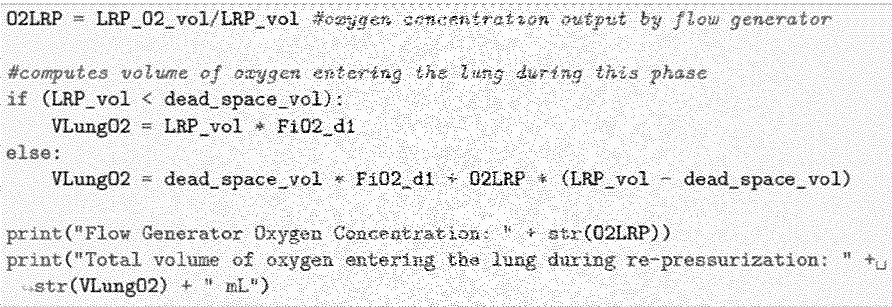
**Flow Generator Oxygen Concentration: 0.5938470627348363**
**Total volume of oxygen entering the lung during re-pressurization:**
   **60.62253333333336 mL**
The dead space is now filled with gas from lung re-pressurization at that same oxygen concentration (unless the dead space is larger than the total volume from this phase of the breath, in which case it is some mix of the previous FiO2 and the lung re-pressurization concentration). However, during EEP, the mask portion fills with the characterized leak concentration from earlier. Thus the dead space oxygen content is computed as follows: 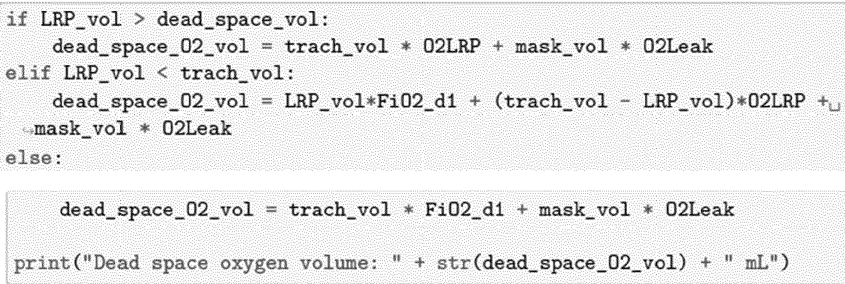 **Dead space oxygen volume: 199.96968275644497 mL**

Next, for the inhalation portion of the breath, the input flow and output flow during inhalation are integrated: 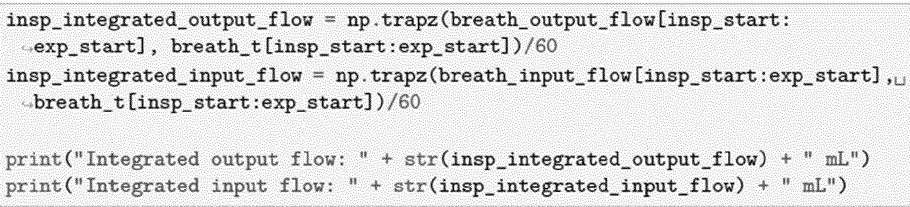
**Integrated output flow: 336.14208333333336 mL**
**Integrated input flow: 99.06675 mL**
Leak volume is obtained by integrating the leak flow over this same period: 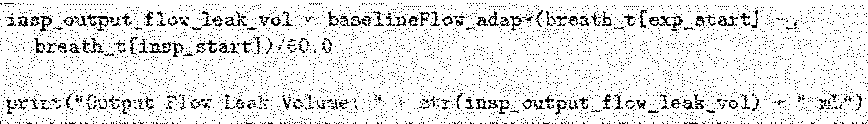 **Output Flow Leak Volume: 19.5375 mL**
The amount of this leak volume that can be accounted for by oxygen is computed the same was as during lung re-pressurization: 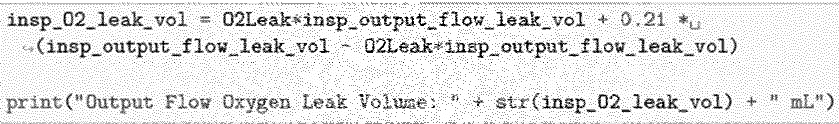 **Output Flow Oxygen Leak Volume: 18.807555173780486 mL**

The tidal volume can be computed as the total output volume subtract the leak volume. The amount of this volume that is oxygen is computed the same way as for the calculaiton of the volume that is oxygen for the lung re-pressurization phase of the breath described above: 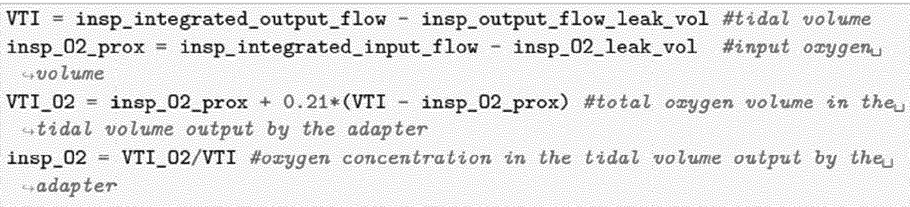 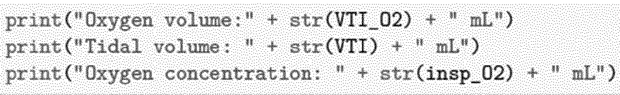
**Oxygen volume:129.8917264127134 mL**
**Tidal volume: 316.6045833333333 mL**
**Oxygen concentration: 0.4102648326981371 mL**

The total volume of oxygen that enters the patient's lung can now be computed. It is dependent on the tidal volume in comparison to the dead space volume as outlined below: 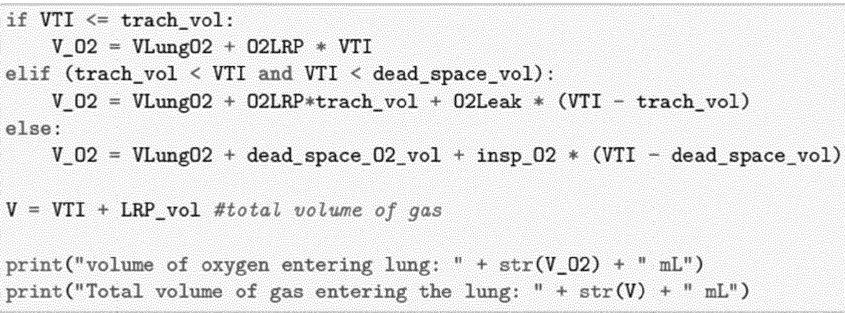
**volume of oxygen entering lung: 287.9177343279575 mL**
**Total volume of gas entering the lung: 392.38275 mL**

Finally, the fraction of inspired oxygen can be computed as the volume of oxygen entering the lung divided by the total volume of gas entering the lung during the entire breath: 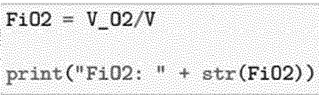 **FiO2: 0.7337675632477664**

Other calculations can also be made based on the patient interface pressures and corresponding oxygen input line flow taken over time. For example, breath timing (such as breath rate, time since start of inhalation, expiratory pause duration, etc.) can be determined from the data collected. These additional calculations can be taken into consideration for the controller to determine the tidal volume of oxygen. Additionally, other input parameters can be measured and added as part of the algorithm to further improve accuracy. Examples of additional input parameters that can be applied to improve the accuracy of flow measurements might include the setting of the oxygen control valve, barometric pressure, size of the patient and other related information.

The description is only exemplary of the principles of the disclosure, and should not be viewed as narrowing the scope of the claims which follow, which claims define the full scope of the invention. Various aspects discussed in one drawing may be present and/or used in conjunction with the embodiment shown in another drawing, and each element shown in multiple drawings may be discussed only once. The described features, structures, or characteristics of configurations of the disclosure may be combined in any suitable manner in one or more configurations. In some cases, detailed description of well-known items or repeated description of substantially the same configurations may be omitted to facilitate the understanding of those skilled in the art by avoiding an unnecessarily redundant description.

Reference in the specification to "one configuration" "one embodiment," "a configuration" "an example," or "an embodiment" means that a particular feature, structure, or characteristic described in connection with the configuration is included in at least one configuration, but is not a requirement that such feature, structure or characteristic be present in any particular configuration unless expressly set forth in the claims as being present. The appearances of the phrase "in one configuration" or "in one example" in various places may not necessarily limit the inclusion of a particular element of the disclosure to a single configuration, rather the element may be included in other or all configurations discussed herein.

As used in this specification and the appended claims, singular forms such as "a," "an," and "the" may include the plural unless the context clearly dictates otherwise. Thus, for example, reference to "a processor" may include one or more of such processors, and reference to "the sensor" may include reference to one or more of such sensors.

As used herein, the term "generally" refers to something that is more of the designated adjective than not, or the converse if used in the negative. As used herein, the term "about" is used to provide flexibility to a numerical range endpoint by providing that a given value may be "a little above" or "a little below" the endpoint while still accomplishing the function associated with the range, for example, "about" may be within 10% of the given number or given range. As used herein, a plurality of items, structural elements, compositional elements, and/or materials may be presented in a common list for convenience. However, these lists should be construed as though each member of the list is individually identified as a separate and unique member.

Numerical data may be expressed in a range format. This range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. For example, a numerical range of "about 5 to about 60" should be interpreted to include not only the explicitly recited values of about 5 to about 60, but also include individual values and sub-ranges within the indicated range. Thus, included in this numerical range are individual values such as 6, 7, 8, 9, etc., through 60, and sub-ranges such as from 10-20, from 30-40, and from 50-60, etc., as well as each number individually. This same principle applies to ranges reciting only one numerical value as a minimum or a maximum. Furthermore, such an interpretation should apply regardless of the breadth of the range or the characteristics being described.

While methods are described herein in discrete steps in a particular order for the sake of clarity, the steps do not require a particular order and more than one step may be performed at the same time. For example, a later step may begin before earlier step completes. Or, a later step may be completed before an earlier step is started. For example, the processor and/or controller can simultaneously determine the flow generator flow output based on patient interface pressure and oxygen input line flow and the leak flow. Additionally, the word "connected" and "coupled" is used throughout for clarity of the description and can include either a direct connection or an indirect connection.

Other embodiments and configurations may be devised which do not depart from the scopes of the claims. Features from different embodiments and configurations may be employed separately or in combination. Accordingly, all additions, deletions and modifications to the disclosed subject matter that fall within the scopes of the claims are to be embraced thereby, as the invention is defined by the claims which follow.

## Claims

1. A system (10) for determining total flow output from a flow generator, the system comprising:
the flow generator (14) for receiving an input of low-flow, high-pressure compressed oxygen and outputting a high-flow, low-pressure gas source, the flow generator (14) for connection to a patient interface (18);
the patient interface (18) in communication with a patient interface pressure sensor (22) to measure a patient interface pressure at the patient interface (18);
an oxygen input line (27) for conveying oxygen from an oxygen source (44) to the flow generator (14), the oxygen input line (27) comprising an oxygen control valve (31) for controlling a flow of oxygen from the oxygen source (44) to the flow generator (14), and an oxygen input line sensor (35) for determining oxygen input line flow;
a controller (40);
wherein
the patient interface pressure sensor (22) and the oxygen input line sensor (35) are in communication with the controller (40),
the system **characterized in that** the flow generator (14) has a plurality of known oxygen flow outputs at a plurality of known oxygen input line pressures and a plurality of known patient interface pressures;
the controller (40) is programmed with at least one algorithm to calculate total flow output based on known patient pressure at the patient interface (22) and known oxygen input line flow, the algorithm being unique for the flow generator (14); and
the controller (40) is programmed to receive a patient interface pressure from the patient interface pressure sensor (122) and an oxygen input line flow from the oxygen input line sensor (35) and determine, based on the algorithm, the total flow output from the flow generator (14) based on the patient interface pressure at the patient interface (18) and the oxygen input line flow.

2. The system of claim 1, wherein the oxygen input line sensor (35) comprises a pressure sensor (37) for measuring oxygen input line pressure and wherein the oxygen input line flow is determined by the oxygen input line pressure.

3. The system of claim 1 or claim 2, wherein the oxygen input line sensor comprises an oxygen flowmeter (39) for measuring an oxygen input line flow.

4. The system of any one of claims 1-3, wherein the controller (40) is programmed to determine the total flow output from the flow generator based on the patient interface pressure at the patient interface (14) and the oxygen input line flow.

5. The system of any one of claims 1-4, wherein the algorithm comprises a polynomial function defined to fit the plurality of known oxygen flow outputs at the plurality of known oxygen input line pressures and the plurality of known patient interface pressures.

6. The system of any one of claims 1-4, wherein the algorithm is a pre-stored lookup table.

7. The system of any one of claims 1-6, wherein the system further comprises a housing (70), the controller (40), the oxygen control valve (31), and the patient interface pressure sensor (22) located within the housing (70), the housing (70) having a coupler to couple to the oxygen source to the housing, and the housing have a receptacle (78); and wherein the flow generator (14) is coupled to a proximal end of the oxygen input line (27), and wherein a distal end (27b) of the oxygen input line (27) comprises a connector (74) to connect to the receptacle (78) of the housing (70), the connector (74) comprising a type-identifier, and the receptacle (78) comprising a reader in communication with the controller (40), the reader to read the type-identifier and convey the type-identifier to the controller (40).

8. The system of any one of claims 1-7, wherein the controller (40) is programmed to receive, during a pause between a patient exhalation and a patient inhalation, the patient pressure at the patient interface and the oxygen input line flow and determine a leak flow output from the flow generator based on the patient pressure at the patient interface and the oxygen input line flow during the pause between the patient exhalation and the patient inhalation.

9. The system of claim 8, wherein the processor is further programmed to receive,
during the patient inhalation, the patient pressure at the patient interface and the oxygen input line flow and determine a total flow output from the flow generator based on the patient pressure at the patient interface and the oxygen input line flow during a patient inhalation; and
wherein the processor is further programmed to calculate a patient flow by subtracting the leak flow output from the total flow output.

10. The system of claim 9, wherein the processor is further programmed to calculate a tidal volume of gas by integrating the patient flow over a single breath.

11. The system of any one of claims 7-10, wherein the oxygen input line sensor for measuring oxygen input line flow is a flow sensor.

12. The system of any one of claims 7-10, wherein the oxygen input line sensor for measuring oxygen input line flow is an oxygen input line pressure sensor (37).

13. The system of claim 11, wherein the controller (40) comprises one or more processors (49) and a memory (55), the algorithm being stored in the memory (55).

14. The system of any one of claims 1-13, wherein the controller (40) is further programmed to calculate a concentration of oxygen in the patient flow to determine a fraction of inhaled oxygen.

15. The system of any one of claims 1-14, wherein the flow generator (14) comprises a type-identifier, and wherein the controller (40) is programmed to receive data relating to the type-identifier of the flow generator.

## Patentansprüche

1. Vorrichtung (10) zur Bestimmung einer Gesamtflussmenge eines Flussgenerators, wobei die Vorrichtung umfasst:
den Flussgenerator (14) zum Empfangen einer Zufuhr von Niedrigfluss-Hochdruckkomprimiertem-Sauerstoff und zum Ausgeben einer Hochfluss-Niederdruck-Gasquelle, wobei der Flussgenerator (14) mit einer Patientenschnittstelle (18) verbindbar ist;
die Patientenschnittstelle (18) in Verbindung mit einem Patientenschnittstellen-Drucksensor (22) zum Messen eines Patientenschnittstellen-Drucks an der Patientenschnittstelle (18) ist;
eine Sauerstoff-Zuführleitung (27) zum Fördern von Sauerstoff von einer Sauerstoffquelle (44) zu dem Flussgenerator (14), wobei die Sauerstoff-Zuführleitung (27) ein Sauerstoff-Regelventil (31) zum Regeln eines Sauerstoffflusses von der Sauerstoffquelle (44) zu dem Flussgenerator (14) und einen Sauerstoff-Zuführleitungs-Sensor (35) zum Bestimmen des Sauerstoff-Zuführleitungs-Flusses umfasst;
eine Steuervorrichtung (40);
wobei der Patientenschnittstellen-Drucksensor (22) und der Sauerstoff-Zuführleitungs-Sensor (35) mit der Steuervorrichtung (40) in Verbindung stehen,
die Vorrichtung ist **dadurch gekennzeichnet, dass** der Flussgenerator (14) eine Mehrzahl an bekannten Sauerstoffflussmengen bei einer Mehrzahl an bekannten Sauerstoff-Zuführleitungs-Drücken und einer Mehrzahl an bekannten Patientenschnittstellen-Drücken aufweist;
die Steuervorrichtung (40) mit mindestens einem Algorithmus programmiert ist, um die Gesamtflussmenge auf der Grundlage des bekannten Patientendrucks an der Patientenschnittstelle (22) und des bekannten Sauerstoff-Zuführleitungs-Flusses zu berechnen, wobei der Algorithmus für den Flussgenerator (14) einzigartig ist; und die Steuervorrichtung (40) programmiert ist, um einen Patientenschnittstellen-Druck von dem Patientenschnittstellen-Drucksensor (122) und einen Sauerstoff-Zuführleitungs-Fluss von dem Sauerstoff-Zuführleitungs-Sensor (35) zu empfangen und auf der Grundlage des Algorithmus die Gesamtflussmenge des Flussgenerators (14) auf der Grundlage des Patientenschnittstellen-Drucks an der Patientenschnittstelle (18) und des Sauerstoff-Zuführleitungs-Flusses zu bestimmen.

2. Vorrichtung nach Anspruch 1, wobei der Sauerstoff-Zuführleitungs-Sensor (35) einen Drucksensor (37) zum Messen des Sauerstoff-Zuführleitungs-Drucks umfasst und wobei der Sauerstoff-Zuführleitungs-Fluss durch den Sauerstoff-Zuführleitungs-Druck bestimmt wird.

3. Vorrichtung nach Anspruch 1 oder 2, wobei der Sauerstoff-Zuführleitungs-Sensor einen Sauerstoff-Durchflussmesser (39) zum Messen eines Sauerstoff-Zuführleitungs-Flusses umfasst.

4. Vorrichtung nach einem der Ansprüche 1 bis 3, wobei die Steuervorrichtung (40) programmiert ist, um die Gesamtflussmenge des Flussgenerators auf der Grundlage des Patientenschnittstellen-Drucks an der Patientenschnittstelle (14) und des Sauerstoff-Zuführleitungs-Flusses zu bestimmen.

5. Vorrichtung nach einem der Ansprüche 1 bis 4, wobei der Algorithmus eine Polynomfunktion umfasst, die definiert ist, um die Mehrzahl bekannter Sauerstoffflussmengen bei der Mehrzahl an bekannter Sauerstoff-Zuführleitungs-Drücke und der Mehrzahl an bekannter Patientenschnittstellen-Drücke anzupassen.

6. Vorrichtung nach einem der Ansprüche 1 bis 4, wobei der Algorithmus eine vorab gespeicherte Nachschlagetabelle ist.

7. Vorrichtung nach einem der Ansprüche 1 bis 6, wobei die Vorrichtung ferner ein Gehäuse (70) umfasst, in dem die Steuervorrichtung (40), das Sauerstoff-Regelventil (31) und den Patientenschnittstellen-Drucksensor (22) angeordnet sind, wobei das Gehäuse (70) einen Anschluss zum Anschließen der Sauerstoffquelle an das Gehäuse aufweist und das Gehäuse eine Aufnahme (78) aufweist; und
wobei der Flussgenerator (14) an ein proximales Ende der Sauerstoff-Zuführleitung (27) angeschlossen ist, und wobei ein distales Ende (27b) der Sauerstoff-Zuführleitung (27) einen Verbinder (74) zum Verbinden mit der Aufnahme (78) des Gehäuses (70) aufweist, wobei der Verbinder (74) eine Typ-Kennzeichnung umfasst und die Aufnahme (78) ein Lesegerät umfasst, das mit der Steuervorrichtung (40) in Verbindung steht, wobei das Lesegerät die Typ-Kennzeichnung liest und die Typ-Kennzeichnung an die Steuervorrichtung (40) übermittelt.

8. Vorrichtung nach einem der Ansprüche 1 bis 7, wobei die Steuervorrichtung (40) programmiert ist, um während einer Pause zwischen einer Ausatmung des Patienten und einer Einatmung des Patienten den Patientendruck an der Patientenschnittstelle und den Sauerstoff-Zuführleitungs-Fluss zu empfangen und eine Leckageflussmenge des Flussgenerators auf der Grundlage des Patientendrucks an der Patientenschnittstelle und des Sauerstoff-Zuführleitungs-Flusses während der Pause zwischen der Ausatmung des Patienten und der Einatmung des Patienten zu bestimmen.

9. Vorrichtung nach Anspruch 8, wobei die Recheneinheit ferner programmiert ist, um während der Einatmung des Patienten den Patientendruck an der Patientenschnittstelle und den Sauerstoff-Zuführleitungs-Fluss zu empfangen und eine Gesamtflussmenge des Flussgenerators auf der Grundlage des Patientendrucks an der Patientenschnittstelle und des Sauerstoff-Zuführleitungs-Flusses während einer Einatmung des Patienten zu bestimmen; und
wobei die Recheneinheit ferner programmiert ist, um einen Patientenfluss zu berechnen, indem die Leckageflussmenge von der Gesamtflussmenge subtrahiert wird.

10. Vorrichtung nach Anspruch 9, wobei die Recheneinheit ferner programmiert ist, um ein Atemzugvolumen von Gas durch Integrieren des Patientenflusses über einen einzelnen Atemzug zu berechnen.

11. Vorrichtung nach einem der Ansprüche 7 bis 10, wobei der Sauerstoff-Zuführleitungs-Sensor zum Messen des Sauerstoff-Zuführleitungs-Flusses ein Flusssensor ist.

12. Vorrichtung nach einem der Ansprüche 7 bis 10, wobei der Sauerstoff-Zuführleitungs-Sensor zum Messen des Sauerstoff-Zuführleitungs-Flusses ein Sauerstoff-Zuführleitungs-Drucksensor (37) ist.

13. Vorrichtung nach Anspruch 11, wobei die Steuervorrichtung (40) eine oder mehrere Recheneinheiten (4) und einen Speicher (55) umfasst, wobei der Algorithmus in dem Speicher (55) gespeichert ist.

14. Vorrichtung nach einem der Ansprüche 1 bis 13, wobei die Steuervorrichtung (40) ferner programmiert ist, um eine Sauerstoffkonzentration in dem Patientenfluss zu berechnen, um einen Anteil des eingeatmeten Sauerstoffs zu bestimmen.

15. Vorrichtung nach einem der Ansprüche 1 bis 14, wobei der Flussgenerator (14) eine Typ-Kennzeichnung umfasst und wobei die Steuervorrichtung (40) programmiert ist, um Daten zu empfangen, die sich auf die Typ-Kennzeichnung des Flussgenerators beziehen.

## Revendications

1. Système (10) pour déterminer une sortie d'écoulement total en provenance d'un générateur d'écoulement, le système comprenant :
le générateur d'écoulement (14) pour recevoir une entrée d'oxygène comprimé haute pression à écoulement faible et pour émettre en sortie une source de gaz basse pression à écoulement élevé, le générateur d'écoulement (14) étant destiné à être connecté sur une interface de patient (18) ;
l'interface de patient (18) qui est en communication avec un capteur de pression d'interface de patient (22) pour mesurer une pression d'interface de patient au niveau de l'interface de patient (18) ;
une ligne d'entrée d'oxygène (27) pour faire cheminer de l'oxygène en provenance d'une source d'oxygène (44) jusqu'au générateur d'écoulement (14), la ligne d'entrée d'oxygène (27) comprenant une soupape de commande et de régulation d'oxygène (31) pour commander et réguler un écoulement d'oxygène depuis la source d'oxygène (44) jusqu'au générateur d'écoulement (14), et un capteur de ligne d'entrée d'oxygène (35) pour déterminer un écoulement de ligne d'entrée d'oxygène ; et
un contrôleur (40) ;
dans lequel :
le capteur de pression d'interface de patient (22) et le capteur de ligne d'entrée d'oxygène (35) sont en communication avec le contrôleur (40),
le système étant **caractérisé en ce que** :
le générateur d'écoulement (14) comporte une pluralité de sorties d'écoulement d'oxygène connues qui sont à une pluralité de pressions de ligne d'entrée d'oxygène connues et à une pluralité de pressions d'interface de patient connues ;
le contrôleur (40) est programmé à l'aide d'au moins un algorithme afin de calculer une sortie d'écoulement total sur la base d'une pression de patient connue au niveau de l'interface de patient (22) et de l'écoulement de ligne d'entrée d'oxygène connu, l'algorithme étant unique pour le générateur d'écoulement (14) ; et
le contrôleur (40) est programmé pour recevoir une pression d'interface de patient en provenance du capteur de pression d'interface de patient (122) et un écoulement de ligne d'entrée d'oxygène en provenance du capteur de ligne d'entrée d'oxygène (35) et pour déterminer, sur la base de l'algorithme, la sortie d'écoulement total en provenance du générateur d'écoulement (14) sur la base de la pression d'interface de patient au niveau de l'interface de patient (18) et de l'écoulement de ligne d'entrée d'oxygène.

2. Système selon la revendication 1, dans lequel le capteur de ligne d'entrée d'oxygène (35) comprend un capteur de pression (37) pour mesurer une pression de ligne d'entrée d'oxygène et dans lequel l'écoulement de ligne d'entrée d'oxygène est déterminé par la pression de ligne d'entrée d'oxygène.

3. Système selon la revendication 1 ou la revendication 2, dans lequel le capteur de ligne d'entrée d'oxygène comprend un débitmètre d'oxygène (39) pour mesurer un écoulement de ligne d'entrée d'oxygène.

4. Système selon l'une quelconque des revendications 1 à 3, dans lequel le contrôleur (40) est programmé pour déterminer la sortie d'écoulement total en provenance du générateur d'écoulement sur la base de la pression d'interface de patient au niveau de l'interface de patient (14) et de l'écoulement de ligne d'entrée d'oxygène.

5. Système selon l'une quelconque des revendications 1 à 4, dans lequel l'algorithme comprend une fonction polynomiale qui est définie de manière à ajuster la pluralité de sorties d'écoulement d'oxygène connues par rapport à la pluralité de pressions de ligne d'entrée d'oxygène connues et par rapport à la pluralité de pressions d'interface de patient connues.

6. Système selon l'une quelconque des revendications 1 à 4, dans lequel l'algorithme est une table de consultation préstockée.

7. Système selon l'une quelconque des revendications 1 à 6, dans lequel :
le système comprend en outre un logement (70), le contrôleur (40), la soupape de commande et de régulation d'oxygène (31) et le capteur de pression d'interface de patient (22) étant localisés à l'intérieur du logement (70), le logement (70) comportant un coupleur pour coupler la source d'oxygène au logement, et le logement comporte un réceptacle (78) ; et dans lequel :
le générateur d'écoulement (14) est couplé à une extrémité proximale de la ligne d'entrée d'oxygène (27), et dans lequel une extrémité distale (27b) de la ligne d'entrée d'oxygène (27) comprend un connecteur (74) pour une connexion sur le réceptacle (78) du logement (70), le connecteur (74) comprenant un identifiant de type, et le réceptacle (78) comprenant un lecteur en communication avec le contrôleur (40), le lecteur ayant pour fonction de lire l'identifiant de type et de transmettre l'identifiant de type au contrôleur (40).

8. Système selon l'une quelconque des revendications 1 à 7, dans lequel le contrôleur (40) est programmé pour recevoir, pendant une pause entre une expiration du patient et une inspiration du patient, la pression de patient au niveau de l'interface de patient et l'écoulement de ligne d'entrée d'oxygène et pour déterminer une sortie d'écoulement de fuite en provenance du générateur d'écoulement sur la base de la pression de patient au niveau de l'interface de patient et de l'écoulement de ligne d'entrée d'oxygène pendant la pause entre l'expiration du patient et l'inspiration du patient.

9. Système selon la revendication 8, dans lequel :
le processeur est en outre programmé pour recevoir, pendant l'inspiration du patient, la pression de patient au niveau de l'interface de patient et l'écoulement de ligne d'entrée d'oxygène et pour déterminer une sortie d'écoulement total en provenance du générateur d'écoulement sur la base de la pression de patient au niveau de l'interface de patient et de l'écoulement de ligne d'entrée d'oxygène pendant une inspiration du patient ; et
dans lequel le processeur est en outre programmé pour calculer un écoulement de patient en soustrayant la sortie d'écoulement de fuite vis-à-vis de la sortie d'écoulement total.

10. Système selon la revendication 9, dans lequel le processeur est en outre programmé pour calculer un volume de gaz respiratoire courant en intégrant l'écoulement de patient sur une unique respiration.

11. Système selon l'une quelconque des revendications 7 à 10, dans lequel le capteur de ligne d'entrée d'oxygène pour mesurer l'écoulement de ligne d'entrée d'oxygène est un capteur d'écoulement.

12. Système selon l'une quelconque des revendications 7 à 10, dans lequel le capteur de ligne d'entrée d'oxygène pour mesurer l'écoulement de ligne d'entrée d'oxygène est un capteur de pression de ligne d'entrée d'oxygène (37).

13. Système selon la revendication 11, dans lequel le contrôleur (40) comprend un ou plusieurs processeurs (49) et une mémoire (55), l'algorithme étant stocké dans la mémoire (55).

14. Système selon l'une quelconque des revendications 1 à 13, dans lequel le contrôleur (40) est en outre programmé pour calculer une concentration en oxygène dans l'écoulement de patient afin de déterminer une fraction d'oxygène inhalé.

15. Système selon l'une quelconque des revendications 1 à 14, dans lequel le générateur d'écoulement (14) comprend un identifiant de type, et dans lequel le contrôleur (40) est programmé pour recevoir des données se rapportant à l'identifiant de type du générateur d'écoulement.
